# EUROPEAN PATENT SPECIFICATION

(11) **EP 4 270 222 B1**
(45) Date of publication and mention of the grant of the patent: **26.03.2025**
(21) Application number: 22170698.9
(22) Date of filing: 29.04.2022
(51) Int. Cl.: G06F 21/32, H04L 9/40, G06V 40/00, A61B 5/00

(54) **DEVICE, SYSTEM AND METHOD FOR AUTHENTICATING A USER OF A SECURITY SCANNER**
VORRICHTUNG, SYSTEM UND VERFAHREN ZUR AUTHENTIFIZIERUNG EINES BENUTZERS EINES SICHERHEITSSCANNERS
DISPOSITIF, SYSTÈME ET PROCÉDÉ D'AUTHENTIFICATION D'UN UTILISATEUR D'UN SCANNER DE SÉCURITÉ

(43) Date of publication of application: 01.11.2023
(73) Proprietor: Rohde & Schwarz GmbH & Co. KG, 81671 München (DE)
(72) Inventor: Evers, Christian, 85551 Heimstetten (DE)
(74) Representative: Novagraaf Group

(56) References cited:
- EP-A1- 3 425 421
- US-A1- 2021 358 244

## Description

The invention relates to the field of security scanners (which can also be called body scanners or full body scanners) and to authenticating a user (i.e., the person who passes through the security scanner) of such. Authenticating is in particular facilitated by a user device (such as a mobile phone) of the user. More specifically, the user is authenticated by comparing information captured by the security scanner by means of electromagnetic radiation with visual information captured by the user device.

A conventional security scanner is typically used in an airport or a public facility to detect an object on or inside a human body for security screening purposes. This allows for checking people for prohibited items without physically removing clothes or making physical contact.

As a conventional security scanner obtains information about the user, which is highly personal and confidential, the scanner is typically operated by authorities.

However, there is the desire to use the information obtained by the security scanner for other paid services and not only for security purposes provided by authorities. Since the use of this information is only intended for the user himself, there is the need for reliably authenticating the user of the security scanner.

EP 3 425 421 A1 discloses a system and method for identifying a biological target using radar sensors.

US 2021/358244 A1 discloses passive multi-factor access control with biometric and wireless capability.

Therefore, the object of the invention is to provide a device for authenticating a user of a security scanner. Moreover, the object is to provide an according method, system and computer program.

The invention is set out in the appended set of claims. The object is solved by the features of the independent claims. The dependent claims contain further developments.

An inventive device for authenticating a user is configured to: compare first data captured by a user device to second data captured by a security scanner; and authenticate the user of the user device, if the first data matches the second data; wherein the second data is captured by the security scanner by means of electromagnetic radiation with a wavelength in a mm range and/or a cm range; the first data comprises visual information of the user of the user device; and the second data comprises biometrical information.

This is beneficial, as data that can be captured by a user of a user device (e.g. a common device like a mobile phone with a camera) can be compared to data captured by a security scanner to authenticate the user of the user device before transmitting confidential data to the user device.

In particular, the user is authenticated before confidential data is transmitted from the security scanner to the user device.

In particular, the user device comprises at least one of: a mobile phone, a mobile phone with a camera, a laptop computer with a camera, a computer with a camera, a tablet computer with a camera.

In particular, the device is configured to determine that the first data matches the second data, if a deviation between the first and second data is below a predefined threshold.

In particular, the user device is external to the device for authenticating a user.

In particular, to compare the first data, the device is configured to receive the first data from the user device.

In particular, the security scanner is external to the device for authenticating a user.

In particular, to compare the second data, the device is configured to receive the second data from the security scanner.

In particular, the device for authenticating a user can also be integrated in the security scanner, or the user device.

In particular, the biometrical information can be obtained by the security scanner. In particular, the biometrical information can be transmitted to the device after it is obtained by the security scanner. In particular, the biometrical information can be obtained by the device itself.

In particular, the user is authenticated before transmitting confidential data to the user takes place.

In particular, the device is a part of the security scanner or is the security scanner.

Advantageously and preferably, the first data comprises a picture of the user.

This is beneficial, as biometrical information obtained from the picture of the user further secures the authentication process.

In particular, the picture of the user comprises a self-portrait (e.g. a "selfie") of the user. In particular, the picture of the user is taken in real time by an app that has access to a camera of the user device.

Using the "selfie" allows for an easy and convenient manner of taking the picture of the user.

Advantageously and preferably, the first data comprises biometrical information, or wherein the device is configured to process the first data to obtain biometrical information.

This ensures that the device can be realized in a versatile manner, which allows to generate the biometrical information in the device itself, or have the biometrical information provided to the device. Thereby, the generation of the biometrical information can be offloaded to the user device, which reduces operating load at the device for authenticating the user.

In particular, if the first data comprises a picture of the user, the biometrical information can be comprised by the picture.

In particular, the biometrical information can be obtained by processing the picture in the user device. In particular, the biometrical information can be obtained by processing the picture in the device.

Advantageously and preferably, the first data and the second data comprise information about a facial area of the user.

This ensures that facial features and lineaments of the user can improve security of the authentication process.

Advantageously and preferably, the device can be configured to ask the user, by means of the user device, to take a certain position and/or gesture (for example ask the user to close one eye). This ensures to reduce the chances that the user is not real and that the device is tricked.

Advantageously and preferably, the device is further configured to transmit additional data to the user device, if the user is authenticated successfully.

This is beneficial as it allows to use all of the information obtained by the security scanner for other purposes than security related ones. The additional data can e.g. be sold to the user, which allows for generating new business models.

In particular, the additional data is data of the security scanner.

In particular, the additional data does not comprise a raw image obtained by the security scanner. This is beneficial because a user should not be aware of the capability of the security scanner, e.g. so that the user cannot figure out ways on how to cheat the security scanner.

In particular, no "naked picture" is transmitted in the additional data.

In particular, the additional data can be generated by the security scanner based on the second data (e.g. based on raw images). In this case, the security scanner can store the additional data (which e.g. comprises non-medical data or medical data) directly to a database.

In particular, the additional data can be generated by the device based on the second data. In this case, the device can store the additional data directly to the database.

In particular, the database can be comprised by the device or the security scanner, or can be external to the device or the security scanner.

Advantageously and preferably, the additional data comprises information regarding the size of a body part of the user.

This is beneficial as the information regarding the size of the body parts can be used for fitting purposes.

In particular, the information regarding the size of a body part of the user comprises at least one of: hip width, arm length, leg length, shoulder width. This ensures that the user may be provided with an exact size of clothes.

In particular, the information regarding the size of a body part of the user can be referred to as non-medical information. This is because it relates only to the size of body parts, but not to diseases associated with said size.

Advantageously and preferably, the additional data comprises a normalized confection size.

This is beneficial, as it makes fitting of clothes even more precise.

In particular, the confection size of the user can be referred to as non-medical information. This is for the same reasons as above.

In particular, the user device is configured to tell the user which confection size the user has for various brands. If the normalized confection size is "50", the user device may tell that for brand A it is 52, or for brand B is "48". This is beneficial as the user knows what size to buy for different brands. The normalized confection size can be determined e.g. by the device, the user device or the security scanner.

Advantageously and preferably, the additional data comprises information regarding a medical anomaly of the body of the user.

This is beneficial as it supports medical check-ups of the user.

In particular, the information regarding a medical anomaly of the user's body can be referred to as medical information.

In particular, the information regarding a medical anomaly may include information regarding at least one of: thicker liver, oblique position of the spine, hip obliquity, thyroid problems.

Advantageously and preferably, the first data further comprises unique information regarding the security scanner and wherein the device is further configured to assess whether the second data is captured by a security scanner which corresponds to the unique information.

This ensures that security of the authentication process is further increased.

In particular, the unique information is a number or a serial number. In particular, the unique information can be included in a QR code. In particular, the unique information can be captured by the user when walking through the security scanner (e.g. by scanning the QR code with the user device). In particular, the authentication is only successful if the first and second data matches AND if the unique information matches with the security scanner which captured the second data.

Advantageously and preferably, the first data comprises additional identification data in the form of a time stamp indicating when the user went through the security scanner, or in the form of identification information of the user, preferably captured by a reader in the area of the security scanner; and wherein the device is configured to only use second data for comparison, which corresponds to the time stamp or the identification information.

This is beneficial as it further increases the security and efficiency of the authentication process.

The time stamp facilitates that a database can be queried more efficiently. A query of the database is simpler, because the time stamp is used when the user walks through the security scanner. Only that kind of second data which is linked to the user's identification information is used for comparison. The data that is captured with the security scanner can be linked to a time stamp and/or identification information. For example, the user walking through the security scanner can put his identification (ID) card into a reader. In particular, the identification information (i.e. ID information, relating to the ID card) is read and linked to the second data captured by the security scanner.

Advantageously and preferably, the device is a cloud computing device.

This is beneficial, as an authentication service can be flexibly provided to a security scanner, without the need for modification or new hardware of the security scanner.

In particular, the device and/or the cloud computing device can be realized by means of a computer program.

An inventive system comprises: the inventive device or any of its advantageous forms described above, and a security scanner, wherein the security scanner is configured to store the second data and in particular the additional data in a database.

This ensures that a security scanner can be equipped with the device for authenticating a user.

In particular, the second data is stored in an encrypted manner. In particular, the additional data can be deleted after a predetermined amount of time (for example 1 week).

Advantageously and preferably, the security scanner is configured to capture the additional data if a control signal is provided to the security scanner.

This ensures that a payment process can be effectively implemented. That is, the control signal can be provided after a payment of a service has been made.

In particular, the control signal includes at least one of: a press of a button (e.g. by the user), a trigger from the system (e.g. released by the user or by a control device, e.g. a payment system that transmits the control signal if the user paid for the service).

Advantageously and preferably, the security scanner is configured to increase the resolution if the control signal is present.

This ensures that the additional data is captured with a higher resolution only, if the control signal is present, as capturing with a higher resolution might take more time.

Advantageously and preferably, the system further comprises an artificial intelligence, AI, module configured to detect a medical anomaly based on the second data.

This is beneficial as it allows for AI based detection of a medical anomaly.

In particular, the AI module is part of the security scanner and/or the system. Input knots of the AI module can be fed with visual data of the additional data (for example pixels). Output knots of the AI module indicate a medical anomaly. The AI module e.g. can be implemented by means of machine learning and/or a neural network.

Advantageously and preferably, the system further comprises the user device in form of at least one of: a mobile phone; a laptop computer; a tablet device; a computer comprising a camera.

This ensures that the authentication of the user can be supported by the user device of the user.

An inventive method for authenticating a user comprises the steps of: comparing, by a device, first data captured by a user device to second data captured by a security scanner; and authenticating, by the device, the user of the user device, if the first data matches the second data; wherein the second data is captured by the security scanner by means of electromagnetic radiation with a wavelength in a mm range and/or a cm range; the first data comprises visual information of the user of the user device; and the second data comprises biometrical information.

Advantageously and preferably, the first data comprises biometrical information, or wherein the method further comprises processing, by the device, the first data to obtain biometrical information.

Advantageously and preferably, the first data and the second data comprise information about a facial area of the user.

Advantageously and preferably, the method further comprises transmitting, by the device, additional data to the user device, if the user is authenticated successfully.

Advantageously and preferably, the additional data comprises information regarding the size of a body part of the user.

Advantageously and preferably, the additional data comprises a normalized confection size.

Advantageously and preferably, the additional data comprises information regarding a medical anomaly of the body of the user.

Advantageously and preferably, the first data further comprises unique information regarding the security scanner and wherein the method further comprises assessing, by the device, whether the second data is captured by a security scanner which corresponds to the unique information.

Advantageously and preferably, the first data comprises additional identification data in the form of a time stamp indicating when the user went through the security scanner, or in the form of identification information of the user, preferably captured by a reader in the area of the security scanner; and wherein the method further comprises only using, by the device, second data for comparison, which corresponds to the time stamp or the identification information.

Advantageously and preferably, the method is a cloud computing method.

The inventive method comprises the same advantages as the inventive device.

An inventive computer program comprises program code for performing steps of the above described inventive method, when the computer program product runs on a computer or a digital signal processor.

The inventive computer program product comprises the same advantages as the inventive device.

An exemplary embodiment of the invention is now further explained with respect to the drawings by way of examples only, in which
- FIG. 1: shows a schematic view of a device according to an embodiment of the present invention;
- FIG. 2: shows a schematic view of a device according to an embodiment of the present invention in more detail;
- FIG. 3: shows a schematic view of data which can be obtained by a security scanner;
- FIG. 4: shows a schematic view of a system according to an embodiment of the present invention; and
- FIG. 5: shows a schematic view of a method according to an embodiment of the present invention.

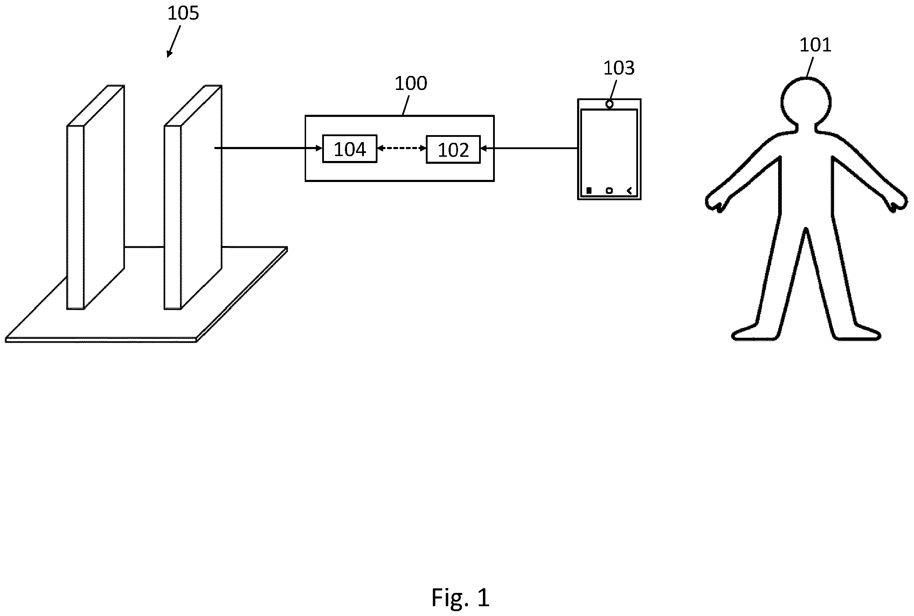

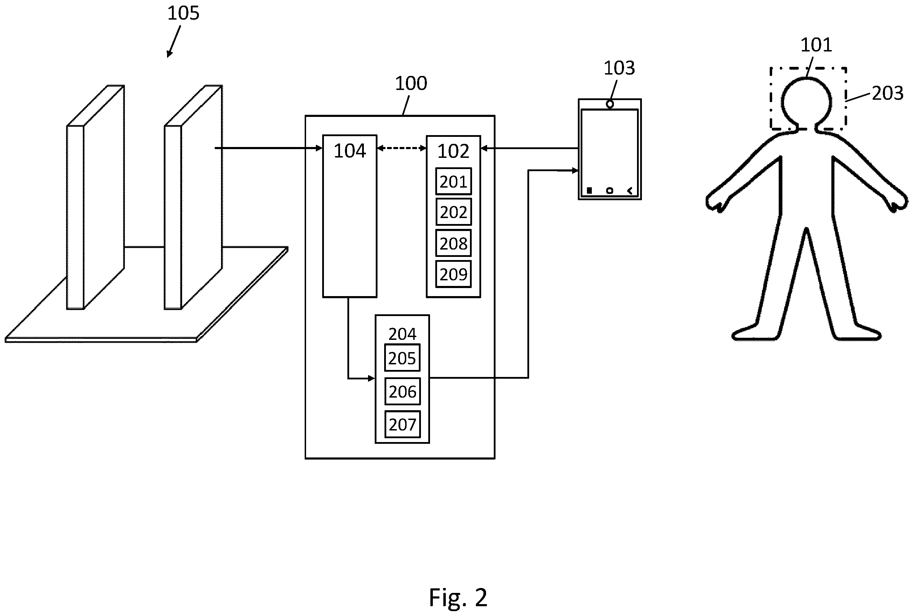

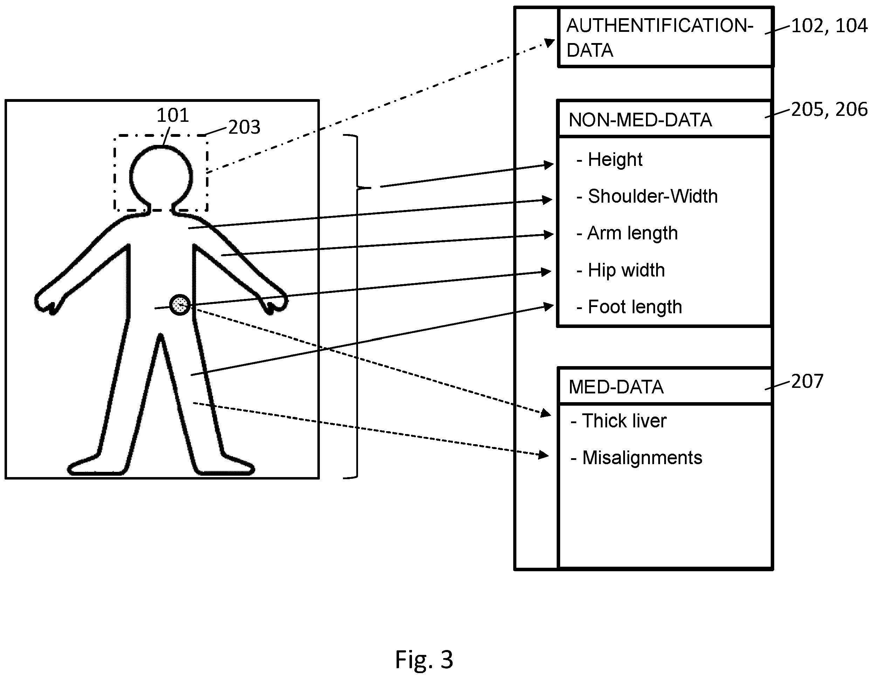

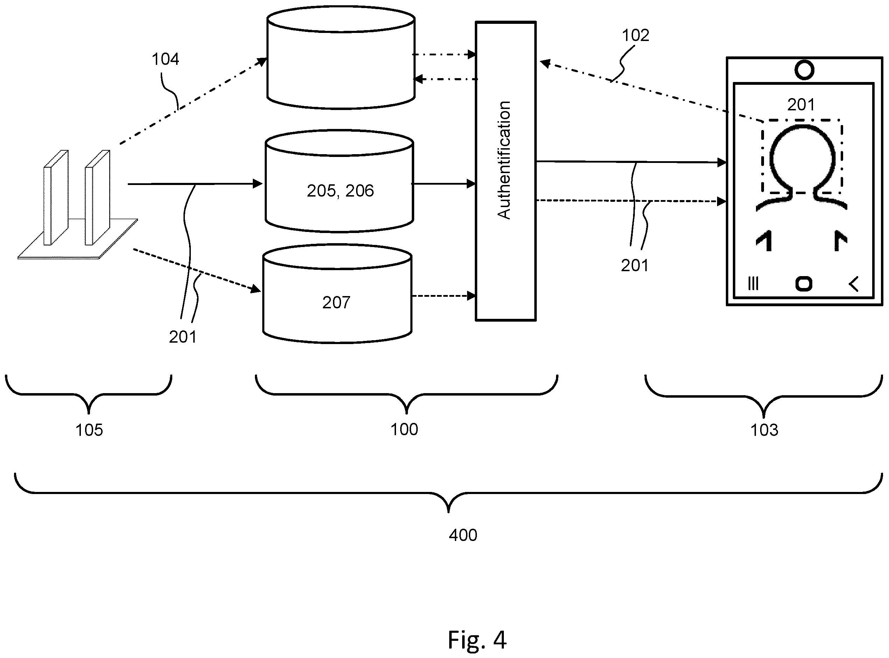

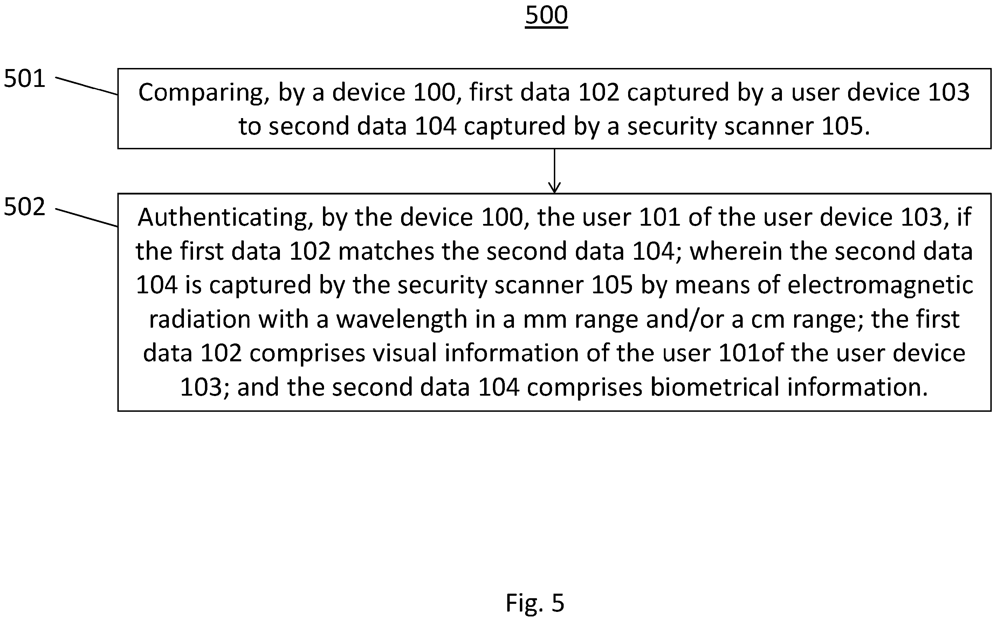

In the following, the function of an embodiment of the inventive device is described based on FIG. 1. Then, based on FIG. 2, the inventive device is described in more detail, in particular by explaining optional features of the device. In FIG. 3, information which can be obtained by a security scanner and, after successful authentication, can be provided to a user device, is explained. In FIG. 4, a system which comprises a security scanner 105, a device 100 for authentication a user and a user device 103 is described. FIG. 5 shows an illustrative view of a method for authenticating a user of a security scanner.

Fig. 1 schematically shows a device 100 for authenticating a user 101 of a security scanner 105. The user 101 is the person who passes through the security scanner 105.

To authenticate the user 101, the device 100 is configured to compare first data 102 captured by a user device 103 to second data 104 captured by a security scanner 105. The user device 103 is e.g. carried by the user 101 when using the security scanner 105.

The user 101 of the user device 103 is authenticated, if the first data 102 matches the second data 104. To obtain the second data 104, the second data 104 is forwarded to the device 100 once it is captured by the security scanner 105 by means of electromagnetic radiation with a wavelength in a mm range and/or a cm range. Comparison of the first and second data is enabled as the first data 102 comprises visual information of the user 101 of the user device 103 and the second data 104 comprises biometrical information.

The security scanner, (which also may be referred to as body scanner or full-body scanner) normally operates in an EHF range (e.g., 30 GHz to 300 GHz). The wavelength ranges from 10 mm to 1 mm. Thus, the radiation in this band can be called cm waves or millimeter waves. However, frequencies below are also possible.

While a conventional security scanner only analyzes whether a prohibited item is hidden in a pocket of the user 101 or not, the device 100 enables to securely share further details of a human body (that is, the body of the user 101), which are also captured by the security scanner 105.

With the strict authentication measures implemented by the device 100, for example airports or facilities themselves (that is, private companies) can operate the security scanner and not only authorities. One scenario could be that travelers who use the security scanner 105 in order to board their flights can receive more details upon request. For example, travelers can pay for additional data.

FIG. 2 shows a schematic view of a device 100 according to an embodiment of the present disclosure in more detail. The device 100 shown in FIG. 2 comprises all features and functionality of the device 100 of FIG. 1, as well as the following optional features:
As illustrated in FIG. 2, the first data 102 optionally can comprise a picture 201 of the user 101. Further optionally, the first data 102 can comprise biometrical information 202. Additionally or alternatively, the device 100 can process the first data 102 to obtain biometrical information 202. The biometrical information 202 can in particular be obtained based on the picture 201 of the user 101. Optionally, the device 100 can correct reflections due to wet skin or makeup which occur in the picture 201.

Further optionally, the first data 102 can comprise information about a facial area 203 of the user 101. This may in particular be the case, if the first data 102 relates to a picture 201 of the face of the user 101, e.g., a "selfie" of the user 101. The biometrical information 202 can in particular be obtained based on the information about a facial area 203 of the user 101.

Further optionally, the second data 104 can comprise information about a facial area 203 of the user 101. The information about the facial area 203 of the user 101 which is comprised in the second data 104 is in particular obtained by a scan performed by the security scanner 105.

Further optionally, the device 100 can be configured to transmit additional data 204 to the user device 103, if the user 101 is authenticated successfully.

In other words, the device 100 enables that only the user 101 who went through the security scanner 105 obtains the captured data (i.e., the additional data 204) and nobody else. This can be achieved in particular by performing the following steps: 1. A user walks through the security scanner. 2. The security scanner captures the second data 104. 3. The user 101 authenticates himself. 4. The user receives the additional data 204 (which may include medical data and/or non-medical data, as described below).

The authentication may be done by comparing data captured by the security scanner (i.e., the second data 104) with data captured by the user 101 (i.e., the first data 102).

For example, the user 101 can create a selfie, wherein biometric features can be extracted from the selfie and can be compared to the data captured by the security scanner 105. If a match is detected, the user can view the medical or non-medical data (i.e., the additional data 204).

As it is illustrated in FIG. 2, the additional data 204 optionally may comprises information 205 regarding the size of a body part of the user 101. As this information is only related to the size of the respective part, but not to a disease of the body or body part, it may also be called non-medical data. The information 205 regarding the size of a body part of the user 101 may include at least one of: height (e.g., without hair, since hair is transparent for electromagnetic wave); shoulder width; hip width; arm length; sizes of body parts; orientation of body parts to each other.

Further optionally, the additional data 204 may comprise a normalized confection size 206, which may also be called non-medical data.

However, the additional data 204 optionally may also comprise information 207 regarding a medical anomaly of the body of the user 101. This kind of information 207 may be called medical data.

The information 207 regarding a medical anomaly of the body of the user 101 may include at least one of: thicker liver; oblique position of the spine; hip obliquity; thyroid problems. These are however just examples to illustrated the function of the device. The information 207 regarding a medical anomaly of the body of the user 101 may comprise any information regarding a disease that is detectable by means of electromagnetic waves.

FIG. 3 schematically illustrates the additional data 204 which may comprise information 205 regarding the size of a body part of the user 101, a normalized confection size 206 of the user 101, or a medical anomaly of the body of the user 101. FIG. 3 also schematically illustrates how the first data 102 and second data 104 can be obtained based on a scan, respectively a picture of the user 101, in particular the facial area 203 of which.

Turning back to FIG. 2, the device 100 can optionally be configured to not provide an actual picture of the body of the user 101 that was obtained by means of the security scanner 105. This is e.g. for the case that, for security reasons, the user 101 should not see the actual resolution of the security scanner 105.

Also, e.g. due to data privacy reasons, the device 100 can be configured to only provide the additional data 204 for a predefined amount of time, e.g. several seconds, minutes, hours, days or weeks.

Further optionally, an additional QR code, which could be obtained directly at the security scanner 105 could be used to harden the authentication and/or to prove whether the user paid for the additional data 204. That is, the first data 102 may further comprises unique information 208 regarding the security scanner 105. The device 100 may assess whether the second data 104 is captured by a security scanner 105 which corresponds to the unique information 208.

As illustrated in FIG. 2, the first data 102 optionally may comprise additional identification data 209 in the form of a time stamp or in the form of identification information of the user 101. The time stamp may indicate when the user 101 went through the security scanner 105. The identification information of the user 101 is preferably captured by a reader in the area of the security scanner 105. The device 100 may only use second data 104 for comparison, which corresponds to the time stamp or the identification information.

Optionally, the device 100 can be a cloud computing device.

FIG. 4 shows a system 400 for authenticating a user of a security scanner 105. As illustrated, the system 400 comprises the device 100 as described in view of FIG. 1 or FIG. 2 above and a security scanner 105. To exchange information with the device 100, the security scanner 105 can be configured to store the second data 104, and in particular the additional data 204, in a database. This may also be the case if the device 100 is a part of the security scanner 105. The device 100 however can also be provided to the security scanner 105 as a cloud computing device.

Optionally, the security scanner 105 can capture the additional data 204 if a control signal is provided to the security scanner 105. The security scanner 105 may also increase its resolution, if the control signal is present. On the one hand, it can be controlled that the additional data is only provided if the user 101 has pay for a certain service. On the other hand, the higher resolution is only asked for, if it is desired by the user (as scanning in higher resolution takes more time and otherwise would increase the duration of a conventional security scan).

The system 400 may also include an artificial intelligence, AI, module to detect a medical anomaly based on the second data 104.

As illustrated in FIG. 4, the system 400 may further comprise the user device 103. The user device 103 may have the form of at least one of: a mobile phone; a laptop computer; a tablet device; a computer comprising a camera.

Further optionally, the user device may run an application which is configured to give the user information about the size needed to buy cloths from certain brands. The app may scale the confection size so that the user 101 knows that his actual size corresponds to size 50 for brand X and to size 48 for brand Y.

FIG. 5 shows a method 500 for authenticating a user 101. As illustrated in FIG. 5, the method 500 comprises the steps of: comparing 501, by a device 100, first data 102 captured by a user device 103 to second data 104 captured by a security scanner 105; and authenticating 502, by the device 100, the user 101 of the user device 103, if the first data 102 matches the second data 104; wherein the second data 104 is captured by the security scanner 105 by means of electromagnetic radiation with a wavelength in a mm range and/or a cm range; the first data 102 comprises visual information of the user 101 of the user device 103; and the second data 104 comprises biometrical information.

It is important to note that the inventive system and method very closely correspond. Therefore, all of the above said regarding the system is also applicable to the method and vice versa. Everything which is described in the description and/or claimed in the claims and/or drawn in the drawings can be combined.

The invention is not limited to the illustrated embodiment. All features described above or features shown in the figures can be combined with each other in any advantageous manner within the scope of the invention.

The invention is specified in the appended claims.

## Claims

1. A device (100) for authenticating a user (101), configured to:
- compare first data (102) captured by a user device (103) to second data (104) captured by a security scanner (105);
- authenticate the user (101) of the user device (103), if the first data (102) matches the second data (104); and
- transmit additional data (204) to the user device (103), if the user (101) is authenticated successfully;
wherein the second data (104) is captured by the security scanner (105) by means of electromagnetic radiation with a wavelength in a mm range and/or a cm range;
wherein the first data (102) comprises visual information of the user (101) of the user device (103);
wherein the second data (104) comprises biometrical information; and
wherein the additional data (204) comprises at least one of: information (205) regarding the size of a body part of the user (101), orientation of body parts to each other, a normalized confection size (206), information (207) regarding a medical anomaly of the body of the user (101), a position of a spine.

2. The device (100) according to claim 1, wherein the first data (102) comprises a picture (201) of the user (101).

3. The device (100) according to claim 1 or 2, wherein the first data (102) comprises biometrical information (202), or wherein the device (100) is configured to process the first data (102) to obtain biometrical information (202).

4. The device (100) according to any one of the preceding claims, wherein the first data (102) and the second data (104) comprise information about a facial area (203) of the user (101).

5. The device (100) according to any one of the preceding claims, wherein the first data (102) further comprises unique information (208) regarding the security scanner (105) and wherein the device (100) is further configured to assess whether the second data (104) is captured by a security scanner (105) which corresponds to the unique information (208).

6. The device (100) according to any one of the preceding claims, wherein the first data (102) comprises additional identification data (209) in the form of:
- a time stamp indicating when the user (101) went through the security scanner (105); and/or
- identification information of the user (101), preferably captured by a reader in the area of the security scanner (105); and
wherein the device (100) is configured to only use second data (104) for comparison, which corresponds to the time stamp and/or the identification information.

7. The device (100) according to any one of the preceding claims, wherein the device (100) is a cloud computing device.

8. A system comprising
- the device (100) according to any one of claims 1 to 7, and
- a security scanner (105),
wherein the security scanner (105) is configured to store the second data (104), and in particular the additional data (204), in a database.

9. The system according to claim 8, wherein the security scanner (105) is configured to capture the additional data (204) if a control signal is provided to the security scanner (105).

10. The system according to claim 8 or 9, wherein the security scanner (105) is configured to increase the resolution if the control signal is present.

11. The system according to any one of claims 8 to 10, further comprising an artificial intelligence, AI, module configured to detect a medical anomaly based on the second data.

12. The system according to any one of claims 8 to 11, further comprising the user device in form of at least one of: a mobile phone; a laptop computer; a tablet device; a computer comprising a camera.

13. A method (500) for authenticating a user (101), wherein the method comprises the steps of:
- comparing (501), by a device (100), first data (102) captured by a user device (103) to second data (104) captured by a security scanner (105);
- authenticating (502), by the device (100), the user (101) of the user device (103), if the first data (102) matches the second data (104); and
- transmitting additional data (204) to the user device (103), if the user (101) is authenticated successfully;
wherein the second data (104) is captured by the security scanner (105) by means of electromagnetic radiation with a wavelength in a mm range and/or a cm range;
wherein the first data (102) comprises visual information of the user (101) of the user device (103);
wherein the second data (104) comprises biometrical information; and
wherein the additional data (204) comprises at least one of: information (205) regarding the size of a body part of the user (101), orientation of body parts to each other, a normalized confection size (206), information (207) regarding a medical anomaly of the body of the user (101), a position of a spine.

14. A computer program comprising instructions which, when the program is executed by a computer, cause the computer to carry out the method according to claim 13.

## Patentansprüche

1. Vorrichtung (100) zum Authentifizieren eines Benutzers (101), das konfiguriert ist zum:
- Vergleichen von ersten Daten (102), die durch eine Benutzervorrichtung (103) erfasst werden, mit zweiten Daten (104), die durch einen Sicherheitsscanner (105) erfasst werden;
- Authentifizieren des Benutzers (101) der Benutzervorrichtung (103), falls die ersten Daten (102) mit den zweiten Daten (104) übereinstimmen; und
- Übertragen zusätzlicher Daten (204) an die Benutzervorrichtung (103), falls der Benutzer (101) erfolgreich authentifiziert wird;
wobei die zweiten Daten (104) durch den Sicherheitsscanner (105) mittels elektromagnetischer Strahlung mit einer Wellenlänge in einem mm-Bereich und/oder einem cm-Bereich erfasst werden;
wobei die ersten Daten (102) visuelle Informationen des Benutzers (101) der Benutzervorrichtung (103) umfassen;
wobei die zweiten Daten (104) biometrische Informationen umfassen; und
wobei die zusätzlichen Daten (204) mindestens eines umfassen von: Informationen (205) bezüglich der Größe eines Körperteils des Benutzers (101), einer Ausrichtung von Körperteilen zueinander, einer normierten Konfektionsgröße (206), Informationen (207) bezüglich einer medizinischen Anomalie des Körpers des Benutzers (101), einer Position einer Wirbelsäule.

2. Vorrichtung (100) nach Anspruch 1, wobei die ersten Daten (102) ein Bild (201) des Benutzers (101) umfassen.

3. Vorrichtung (100) nach Anspruch 1 oder 2, wobei die ersten Daten (102) biometrische Informationen (202) umfassen oder wobei die Vorrichtung (100) konfiguriert ist, um die ersten Daten (102) zu verarbeiten, um biometrische Informationen (202) zu erhalten.

4. Vorrichtung (100) nach einem der vorstehenden Ansprüche, wobei die ersten Daten (102) und die zweiten Daten (104) Informationen über einen Gesichtsbereich (203) des Benutzers (101) umfassen.

5. Vorrichtung (100) nach einem der vorstehenden Ansprüche, wobei die ersten Daten (102) ferner eindeutige Informationen (208) bezüglich des Sicherheitsscanners (105) umfassen und wobei die Vorrichtung (100) ferner konfiguriert ist, um zu beurteilen, ob die zweiten Daten (104) durch einen Sicherheitsscanner (105) erfasst werden, der den eindeutigen Informationen (208) entspricht.

6. Vorrichtung (100) nach einem der vorstehenden Ansprüche, wobei die ersten Daten (102) zusätzliche Identifikationsdaten (209) in der Form umfassen von:
- einem Zeitstempel, der angibt, wann der Benutzer (101) den Sicherheitsscanner (105) passiert hat; und/oder
- Identifikationsinformationen des Benutzers (101), die vorzugsweise durch ein Lesegerät in dem Bereich des Sicherheitsscanners (105) erfasst werden; und
wobei die Vorrichtung (100) konfiguriert ist, um für einen Vergleich ausschließlich zweite Daten (104) zu verwenden, die dem Zeitstempel und/oder den Identifikationsinformationen entsprechen.

7. Vorrichtung (100) nach einem der vorstehenden Ansprüche, wobei die Vorrichtung (100) eine Cloud-Computing-Vorrichtung ist.

8. System, umfassend
- die Vorrichtung (100) nach einem der Ansprüche 1 bis 7, und
- einen Sicherheitsscanner (105),
wobei der Sicherheitsscanner (105) konfiguriert ist, um die zweiten Daten (104) und insbesondere die zusätzlichen Daten (204) in einer Datenbank zu speichern.

9. System nach Anspruch 8, wobei der Sicherheitsscanner (105) konfiguriert ist, um die zusätzlichen Daten (204) zu erfassen, falls dem Sicherheitsscanner (105) ein Steuersignal bereitgestellt wird.

10. System nach Anspruch 8 oder 9, wobei der Sicherheitsscanner (105) konfiguriert ist, um die Auflösung zu erhöhen, falls das Steuersignal vorhanden ist.

11. System nach einem der Ansprüche 8 bis 10, ferner umfassend ein Modul für künstliche Intelligenz, KI, das konfiguriert ist, um basierend auf den zweiten Daten eine medizinische Anomalie zu erkennen.

12. System nach einem der Ansprüche 8 bis 11, ferner umfassend die Benutzervorrichtung in Form von mindestens einem von: einem Mobiltelefon; einem Laptop-Computer; einer Tablet-Vorrichtung; einem Computer, umfassend eine Kamera.

13. Verfahren (500) zum Authentifizieren eines Benutzers (101), wobei das Verfahren die Schritte umfasst:
- Vergleichen (501), durch eine Vorrichtung (100), von ersten Daten (102), die durch eine Benutzervorrichtung (103) erfasst werden, mit zweiten Daten (104), die durch einen Sicherheitsscanner (105) erfasst werden;
- Authentifizieren (502), durch die Vorrichtung (100), des Benutzers (101) der Benutzervorrichtung (103), falls die ersten Daten (102) mit den zweiten Daten (104) übereinstimmen; und
- Übertragen zusätzlicher Daten (204) an die Benutzervorrichtung (103), falls der Benutzer (101) erfolgreich authentifiziert wird; wobei die zweiten Daten (104) durch den Sicherheitsscanner (105) mittels elektromagnetischer Strahlung mit einer Wellenlänge in einem mm-Bereich und/oder einem cm-Bereich erfasst werden;
wobei die ersten Daten (102) visuelle Informationen des Benutzers (101) der Benutzervorrichtung (103) umfassen;
wobei die zweiten Daten (104) biometrische Informationen umfassen; und
wobei die zusätzlichen Daten (204) mindestens eines umfassen von: Informationen (205) bezüglich der Größe eines Körperteils des Benutzers (101), einer Ausrichtung von Körperteilen zueinander, einer normierten Konfektionsgröße (206), Informationen (207) bezüglich einer medizinischen Anomalie des Körpers des Benutzers (101), einer Position einer Wirbelsäule.

14. Computerprogramm, umfassend Anweisungen, die, wenn das Programm durch einen Computer ausgeführt wird, den Computer veranlassen, das Verfahren nach Anspruch 13 vorzunehmen.

## Revendications

1. Dispositif (100) permettant d'authentifier un utilisateur (101), configuré pour :
- comparer des premières données (102) capturées par un dispositif utilisateur (103) à des secondes données (104) capturées par un scanner de sécurité (105) ;
- authentifier l'utilisateur (101) du dispositif utilisateur (103), si les premières données (102) concordent avec les secondes données (104) ; et
- transmettre des données supplémentaires (204) au dispositif utilisateur (103), si l'utilisateur (101) est authentifié avec succès ;
dans lequel les secondes données (104) sont capturées par le scanner de sécurité (105) au moyen d'un rayonnement électromagnétique avec une longueur d'onde dans une plage millimétrique et/ou une plage centimétrique ;
dans lequel les premières données (102) comprennent des informations visuelles de l'utilisateur (101) du dispositif utilisateur (103) ;
dans lequel les secondes données (104) comprennent des informations biométriques ; et
dans lequel les données supplémentaires (204) comprennent au moins l'une parmi : des informations (205) concernant la taille d'une partie du corps de l'utilisateur (101), une orientation de parties du corps les unes par rapport aux autres, une taille de confection normalisée (206), des informations (207) concernant une anomalie médicale du corps de l'utilisateur (101), une position d'une colonne vertébrale.

2. Dispositif (100) selon la revendication 1, dans lequel les premières données (102) comprennent une photo (201) de l'utilisateur (101).

3. Dispositif (100) selon la revendication 1 ou 2, dans lequel les premières données (102) comprennent des informations biométriques (202), ou dans lequel le dispositif (100) est configuré pour traiter les premières données (102) pour obtenir des informations biométriques (202).

4. Dispositif (100) selon l'une quelconque des revendications précédentes, dans lequel les premières données (102) et les secondes données (104) comprennent des informations concernant une zone du visage (203) de l'utilisateur (101).

5. Dispositif (100) selon l'une quelconque des revendications précédentes, dans lequel les premières données (102) comprennent en outre des informations uniques (208) concernant le scanner de sécurité (105) et dans lequel le dispositif (100) est configuré en outre pour évaluer si les secondes données (104) sont capturées par un scanner de sécurité (105) qui correspond aux informations uniques (208).

6. Dispositif (100) selon l'une quelconque des revendications précédentes, dans lequel les premières données (102) comprennent des données d'identification supplémentaires (209) sous la forme de :
- un horodatage indiquant le moment où l'utilisateur (101) a traversé le scanner de sécurité (105) ; et/ou
- des informations d'identification de l'utilisateur (101), capturées de préférence par un lecteur dans la zone du scanner de sécurité (105) ; et
dans lequel le dispositif (100) est configuré pour utiliser uniquement les secondes données (104) pour comparaison, qui correspondent à l'horodatage et/ou aux informations d'identification.

7. Dispositif (100) selon l'une quelconque des revendications précédentes, dans lequel le dispositif (100) est un dispositif informatique en nuage.

8. Système comprenant
- le dispositif (100) selon l'une quelconque des revendications 1 à 7, et
- un scanner de sécurité (105),
dans lequel le scanner de sécurité (105) est configuré pour stocker les secondes données (104), et en particulier les données supplémentaires (204), dans une base de données.

9. Système selon la revendication 8, dans lequel le scanner de sécurité (105) est configuré pour capturer les données supplémentaires (204) si un signal de commande est fourni au scanner de sécurité (105).

10. Système selon la revendication 8 ou 9, dans lequel le scanner de sécurité (105) est configuré pour augmenter la résolution si le signal de commande est présent.

11. Système selon l'une quelconque des revendications 8 à 10, comprenant en outre un module d'intelligence artificielle, AI, configuré pour détecter une anomalie médicale en fonction des secondes données.

12. Système selon l'une quelconque des revendications 8 à 11, comprenant en outre le dispositif utilisateur sous forme d'au moins l'un parmi : un téléphone mobile ; un ordinateur portable ; une tablette ; un ordinateur comprenant une caméra.

13. Procédé (500) permettant d'authentifier un utilisateur (101), dans lequel le procédé comprend les étapes consistant à :
- comparer (501), par un dispositif (100), des premières données (102) capturées par un dispositif utilisateur (103) à des secondes données (104) capturées par un scanner de sécurité (105) ;
- authentifier (502), par le dispositif (100), l'utilisateur (101) du dispositif utilisateur (103), si les premières données (102) concordent avec les secondes données (104) ; et
- transmettre des données supplémentaires (204) au dispositif utilisateur (103), si l'utilisateur (101) est authentifié avec succès ; dans lequel les secondes données (104) sont capturées par le scanner de sécurité (105) au moyen d'un rayonnement électromagnétique avec une longueur d'onde dans une plage millimétrique et/ou une plage centimétrique ;
dans lequel les premières données (102) comprennent des informations visuelles de l'utilisateur (101) du dispositif utilisateur (103) ;
dans lequel les secondes données (104) comprennent des informations biométriques ; et
dans lequel les données supplémentaires (204) comprennent au moins l'une parmi : des informations (205) concernant la taille d'une partie du corps de l'utilisateur (101), une orientation de parties du corps les unes par rapport aux autres, une taille de confection normalisée (206), des informations (207) concernant une anomalie médicale du corps de l'utilisateur (101), une position d'une colonne vertébrale.

14. Programme d'ordinateur comprenant des instructions qui, lorsque le programme est exécuté par un ordinateur, amènent l'ordinateur à effectuer le procédé selon la revendication 13.
